# EUROPEAN PATENT APPLICATION

(11) **EP 3 588 084 A1**
(43) Date of publication of application: **01.01.2020**
(21) Application number: 18179366.2
(22) Date of filing: 22.06.2018
(51) Int. Cl.: G01N 33/28, G01N 33/30, G01N 1/14

(54) **MOBILE MAINTENANCE DEVICE AND A METHOD OF MOBILE MAINTENANCE SURVEY**

(71) Applicant: Brisco Systems GmbH, 8335 Hittnau (CH)
(72) Inventor: GOSCH, Jürgen, 8335 Hittnau (CH)
(74) Representative: Troesch Scheidegger Werner AG

(57) **Abstract**

A method of mobile maintenance survey for production sites with lubricant storage tanks is provided. The method comprises flexibly selecting one out of more than one lubricant storage tanks, identifying the lubricant in the selected storage tank, machine-selecting a data set corresponding to said identified lubricant, machine-determining at least one actual value of a parameter of said identified lubricant, comparing said at least one actual value of said parameter of said identified lubricant with at least one corresponding desired value of said parameter of said identified lubricant from the data set, and generating, depending on a result of said comparing, status information of said identified lubricant. Furthermore, a mobile maintenance device is provided.

## Description

The present invention is directed to a method of mobile maintenance survey for production sites with lubricant storage tanks and to a mobile maintenance device.

Lubricants are, inter alia, used to minimize friction and wear and tear but also to cool and damp vibration. Lubricants play, for instance, in the field of industrial metal processing a significant role, in particular for cutting processes such as milling, turning, threading and the like. In these cases, cooling lubricants are often used. Cooling lubricants are usually emulsions consisting of water and a certain amount of lubricant concentrate. Said emulsion is again and again recycled back into the cutting process by circulating pumps. Such a recycling, however, has an impact on the quality of the lubricant. On the one hand, the composition of the lubricant is changed by, e.g., vaporization of water and thus increasing the concentration of the lubricant concentrate. On the other hand, impurities can be introduced, or bacteria can settle and multiply in the storage tank. In case the composition of the lubricant is no longer at its optimum, a negative impact on, inter alia, the tool life and thus increasing operating costs are the consequence. In addition, the surface quality and thus the quality of the product can suffer from an aged or not optimal composed lubricant. To keep such negative impacts to a minimum or avoid them in total, monitoring the lubricant is inevitable. For said purpose, a permanent measurement device is usually installed next to the storage tank of the lubricant to be monitored. However, such permanent measurement devices are rather beneficial for production sites with a large production volume and less interesting for small and medium sized enterprises with smaller storage tanks due to a smaller production volume.

The object of the present invention is to provide a method of mobile maintenance survey for production sites with lubricant storage tanks. A further aspect of the invention addresses the provision of a mobile measurement device for the mobile maintenance survey for production sites with lubricant storage tanks.

This object is achieved by a method according to claim 1.

The method of mobile maintenance survey for production sites with lubricant storage tanks according to the invention comprises flexibly selecting one out of more than one lubricant storage tanks to be surveyed, identifying the lubricant in the selected storage tank, machine-selecting a data set corresponding to said identified lubricant out of a multitude of data sets corresponding to respective different lubricants, each data set comprising at least one desired value of a parameter of the respective lubricant, machine-determining at least one actual value of a parameter of said identified lubricant, comparing said at least one actual value of said parameter of said identified

lubricant with said at least one corresponding desired value of said parameter of said identified lubricant, and generating, depending on a result of said comparing, status information of said identified lubricant.

In case one or more lubricant storage tanks out of a multitude of lubricant storage tanks shall be surveyed, it can be beneficial to apply a method of mobile maintenance survey. In particular, when the use of a method requiring a fixed installed maintenance device at each storage tank is too expensive or economically not feasible. In a potential first step the storage tank to be survey is flexibly chosen out of the multitude of lubricant storage tanks. The selection can, e.g., be made by man, i.e. by an applicant, or by a computer comprising a survey schedule. The term "flexibly" indicates that there is no limitation in the choice or rather nothing limiting the choice. For instance, when a fixed installed maintenance device is used, the actual selection has been made when the device got installed. When applying a method based on such a fixed installed maintenance device, it is always the lubricant tank the device belongs to that is selected. No other storage tank can be selected and thus no "flexibly selecting" is involved. But with the method according to the invention there is the possibility to pick any of the storage tanks. Therefore, the selection is fully flexible. When the selection is made, the lubricant in the selected storage tank is identified. With the precise knowledge of the content of the storage tank, it is possible to machine-select, e.g., by a computer, a data set that corresponds to the identified lubricant. The data set is picked out of a multitude of data sets. Data sets that are not picked belong to specific lubricants differing from the identified one. Each of the data sets comprise at least one desired value of a parameter of the respective lubricant, such as a desired pH value, a desired temperature, a desired refraction index and so on. After at least one actual value of a parameter of the identified lubricant has been machine-determined, e.g., by means of a pH meter, a thermometer, a refractometer and so on, at least one actual value of a parameters and of a corresponding desired value of a parameter are compared to each other. "Corresponding" in this context means, for instance, that the values belong to the same kind or category of parameters, e.g., an actual refraction index of the identified lubricant is compared to a desired refraction index of the same lubricant. Based on the comparison, status information of the identified lubricant is generated. A status information can, e.g., be the difference of the values of one or more parameters. In case the desired value of a parameter is not just a specific single value but is a range or a specific value comprising a tolerance level, the status information may indicate whether the actual value of this parameter is within the range or tolerance level. The status information can also indicate whether the actual value of the parameter is below or above the desired value.

However, the method is not limited to the comparison of one desired value to one actual value of the same parameter, e.g., the comparison of one actual pH value to one desired pH value. It is also possible to compare single actual and single desired values of a multitude of parameters, e.g., compare an actual pH value to a desired pH value and in addition an actual concentration value to a desired concentration value and so on.

Furthermore, it is also possible to compare a series of desired values of a parameter to a series of actual values of the same parameter. For instance, the data set belonging to the identified lubricant can comprise a sequence of several desired pH values representing a desired pH trend, e.g., over a certain period. The several actual pH values representing an actual pH trend, e.g., over a certain period, can either all be instantly determined by the step of machine-determining at least one actual value of a parameter of said identified lubricant or can be generated by combining, for instance, a single pH value determined instantly by machine-determining at least one actual value of a parameter of said identified lubricant with pH values that were machine-determined in the past. Actual values of parameters that were determined in the past and are intended to contribute to an actual parameter trend can, e.g., also be part of the data set belonging to the identified lubricant. The comparing is thus a comparison of an actual parameter (e.g. pH) trend or time series to a desired parameter (e.g. pH) trend or time series. Such trend comparisons can of course be applied to one or more parameters. The status information is consequently generated depending on a result of said comparing. Sometimes a comparison of a trend is more meaningful than the comparison of single values. For instance, the decrease of a pH value over time can be an indication for bacteria growth.

Moreover, the actual and the desired value of a parameter can be more than just a simple measurand. For instance, the (desired or actual) value of a parameter can be generated by setting values of more than one measurand (pH, temperature, density, ...) in relation to each other in order to provide an interrelated combination. For instance, if a sum of the numerical value of the density and the numerical pH value should be between 6 and 8, the desired value is 6 to 8 and the parameter is defined as the numerical sum of density and pH value. In order to generate the corresponding actual value of the parameter, both the pH value and the density of the lubricant are machine-determined and added up before the comparison of the actual value and the desired value of the parameter is performed.

Nonetheless, it is also possible that the parameter is a rate of change, e.g. per time unit. For instance, a certain shift of the pH value over time is acceptable, however, if the pH value decreases too rapidly, this might be an indicator for a bacterial contamination. Therefore, the desired value of the pH change over time can be set to, e.g., 0.2/month. In order to generate the corresponding actual value of the parameter, i.e. the actual change rate of the pH, an instantly machine-determined actual pH value is set into relation to at least one actual pH value determined in the past. Based on these at least two values the rate of change of the pH value and thus the actual value of the parameter to be compared with the desired value of the parameter is determined.

The various natures of the parameters (single measurand, time series, interrelated combination of measurands, rate of change etc.) can be arbitrarily combined and applied to all kinds of measurands. It is also possible that a specific measurand, e.g. the temperature, is surveyed by a combination, e.g., the single measurand, e.g. the actual temperature, and its change over time, e.g. the actual temperature change rate, are observed and compared to the corresponding desired values, e.g. the desired temperature and desired temperature change rate respectively.

In one embodiment of the method according to the invention, which may be combined with any of the preaddressed embodiments and any of the embodiments still to be addressed unless in contradiction, the parameter comprises pH, conductivity, density, temperature, viscosity, absorption, refractive index, total hardness, concentration of the lubricant concentrate, nitrite concentration, nitrate concentration, chloride concentration, metal concentration, in particular cobalt concentration and/or beryllium concentration, and/or bacteria count. Furthermore, the at least one parameter can comprise a time series of one of the above identified measurands, a change rate of one of the above identified measurands and/or an interrelated combination of more than one of the above identified measurands.
Various parameters can be relevant for the mobile maintenance survey of lubricants in storage tanks. For instance, the survey of the concentration of the lubricant concentrate in the storage tank is important to enable a long life time and high production quality of the machine being supplied with the lubricant. The survey of the pH may be helpful to realize that the lubricant is, e.g., contaminated with bacteria diminishing the quality of the lubricant, too low attacking the tools and/or the work pieces, too high indicating foreign matter and causing skin irritation and so on. An increased conductivity may be an indication for a destabilization of the lubricant and/or for a contamination of or depositions on the tool. The survey of other parameters such as nitrite concentration and/or nitrate concentration might be helpful when it comes to an environmentally compatible disposal of the lubricant. The survey of the concentration of metals being present in the work pieces is helpful to identify and limit a health risk caused by metals such as cobalt and beryllium. In case the viscosity of the lubricant is not within the tolerance range anymore, further use of the lubricant can do harm to the tools that gets lubricated by said lubricant. Depending on the customized requirement, it might be enough to survey only one parameter. However, the survey of several parameters provides mostly for a more reliable survey. A preferred combination of parameters comprises at least the pH and the concentration of the lubricant concentrate. A further preferred combination comprises in addition concentration parameters such as nitrite and/or nitrate. The optimum operating parameters vary from lubricant to lubricant. Examples for such operating parameters in dependency of their utilization are given in the following table:

**CFU: Colony Forming Unit**

| *Parameter* | *Unit* | *Metal cutting* | *Metal forming* |
|---|---|---|---|
| pH | / | ca. 7 | ca. 7 |
| Conductivity | [µS/cm] | max. 1000-1500 | max. 50-75 |
| Total hardness | [°d] | 5-20 | max. 15 |
| Nitrite | [ppm] | max. 5 | max. 5 |
| Nitrate | [ppm] | max. 50 | max. 50 |
| Chloride | [ppm] | max. 250 | max. 50 |
| Bacteria count | [CFU/ml] | max. 102 | max. 10² |

In one embodiment of the method according to the invention, which may be combined with any of the preaddressed embodiments and any of the embodiments still to be addressed unless in contradiction, the method further comprises displaying the need of at least one additive in dependency of said status information and/or automatically adding at least one additive to said identified lubricant in the selected storage tank in dependency of said status information.

If, for example, the status information depending on the comparison of the actual concentration of lubricant concentrate to the corresponding desired concentration indicates that the actual concentration is too low, the need of additional concentrate can be displayed, e.g., on a display and an applicant can thus add some lubricant concentrate to the storage tank. However, it is also possible that the missing concentrate is automatically added to the storage tank by means of, e.g., a filling unit or the like, with or without displaying the need of concentrate. The status information may also indicate a need of other additives such as emulsifiers, corrosion inhibitors, polar lubricants, sulfur additives, phosphor additives, anti-foaming agents, biocidal products, solubilizers, antioxidants, stabilizers, colorants, fragrances, non-ferrous metal inhibitors and so on. These additives can also be manually or automatically added.

In one embodiment of the method according to the invention, which may be combined with any of the preaddressed embodiments and any of the embodiments still to be addressed unless in contradiction, the additive is at least one of a lubricant, of a lubricant concentrate, of water, and of a mixture thereof.
For instance, in case the status information indicates that the actual concentration of lubricant concentrate is too low, concentrate is added. In case the status information indicates that the actual concentration is too high, water is added in order to dilute. In case the status information indicates that the salt concentration is too high, it might be helpful to add new lubricant of the right mixture of lubricant concentrate and water. However, if the status information indicates that the number of bacteria is too high, it might also be advisable to add, e.g., antibacterial substances.

In one embodiment of the method according to the invention, which may be combined with any of the preaddressed embodiments and any of the embodiments still to be addressed unless in contradiction, the method further comprises machine-determining the filling level of said identified lubricant in the selected storage tank and filling the storage tank in dependency of the filling level.

The machine-determination of the filling level is helpful to ensure an amount of lubricant big enough to reliably lubricate the machine or tools to be lubricated. For instance, the addition of, e.g., lubricant, lubricant concentrate and/or water must not necessarily be triggered by a status information based on a parameter such as a pH value, a lubricant concentrate concentration, a refraction index or the like, but can be triggered instead by a machine-based determination of the filling level of the lubricant in the storage tank to be surveyed. However, it is also possible to trigger the addition of at least one additive by both the actual filling level and status information depending on the comparison of at least one actual value of a parameter with at least one corresponding desired value of a parameter. In case, e.g., the concentration of the lubricant concentration is too high, and the storage tank is partially empty, either a computer or an applicant can calculate depending on the given information the amount of water and lubricant concentrate to be added to both fill the storage tank to its maximum filling level and to gain the desired lubricant concentrate concentration in the storage tank. The determination of the filling level triggering the addition of an additive can comprise, e.g., the continuous monitoring of the filling level (e.g. by means of a linear filling level detector) in combination with a defined start level and stop level. As soon as a filling level is determined that is equal to the defined start level, the addition starts. As soon as a filling level is determined that is equal to the defined stop level, the addition stops. It is also possible that the determination of the filling level triggering the addition of an additive comprises, e.g., solely the determination of a minimum level. As soon as the filling level reaches the minimum level, a defined volume of additive that will fit into the storage tank is added. Alternatively, the volume can be regulated via a defined time and a defined or known flow rate. In another example, the determination of the filling level triggering the addition of an additive can comprise, e.g., the sole determination of a maximum level: a small amount of additive is constantly added until the maximum level is reached.

In one embodiment of the method according to the invention, which may be combined with any of the preaddressed embodiments and any of the embodiments still to be addressed unless in contradiction, machine-determining of at least one actual value of a parameter of said identified lubricant comprises performing a measurement.

In order to machine-determine at least one actual value of a parameter of the identified lubricant, a measurement can be performed. Such a measurement can, e.g., be conducted with a pH meter, a refractometer, a thermometer and the like.

In one embodiment of the method according to the invention, which may be combined with any of the preaddressed embodiments and any of the embodiments still to be addressed unless in contradiction, the method further comprises rinsing a measurement path for said measurement with a rinsing liquid comprising at least one of water and of a cleaning agent.

In order to machine-determine actual values of parameters of various lubricants differing from each other, cross contamination between the lubricants should be avoided. By rinsing the measurement path along which the lubricant is led for the measurement by water and/or a cleaning agent, the risk of cross contamination is minimized or even fully prevented. The measurement path can, e.g., be rinsed alternating by water and one or more cleaning agents. Such an alternation may be conducted various times, e.g., one, two, three or even four times. In some examples a first and a last rinsing is conducted with water.

In one embodiment of the method according to the invention, which may be combined with any of the preaddressed embodiments and any of the embodiments still to be addressed unless in contradiction, the rinsing is performed before and/or after flexibly selecting one out of more than one lubricant storage tanks.

In general, the rinsing preventing cross contamination can be performed before a further lubricant storage tank is flexibly selected, after a further lubricant storage tank is flexibly selected or before and after. Whenever the rinsing is performed, it should be done before the machine-determination of an actual value of a parameter of the further lubricant.

In one embodiment of the method according to the invention, which may be combined with any of the preaddressed embodiments and any of the embodiments still to be addressed unless in contradiction, the method further comprises performing a test measurement with a reference substance, before said machine-determining of at least one actual value of a parameter of said identified lubricant.

To make sure that, e.g., the measurement path is not still contaminated with a lubricant measured beforehand, the performance of a test measurement can be advisable. For instance, a refraction measurement with water as reference substance can be performed. The actual determined refraction index can be compared with a desired refraction index. In case the actual and the desired values are in line, the measurement path is free from lubricant of a former measurement. However, such a test measurement can also be used to check if the hardware (e.g. sensors and so on) and/or the software (e.g. evaluation software) used for machine-determining at least one actual value of a parameter of the identified lubricant, work faultless. For instance, a pH measurement with water as reference substance can be performed. The actual determined pH value can be compared with a desired pH value. In case the actual and the desired values are in line, the pH meter works properly.

In one embodiment of the method according to the invention, which may be combined with any of the preaddressed embodiments and any of the embodiments still to be addressed unless in contradiction, the method further comprises performing a test measurement with a reference substance, before said machine-determining of at least one actual value of a parameter of said identified lubricant. Furthermore, the machine-determining comprises performing a measurement and rinsing a measurement path for said measurement with a rinsing liquid comprising at least one of water and of a cleaning agent. The rinsing is performed in dependency of a result of said test measurement.

In case the test measurement indicates a contamination of the measurement path, the rinsing of the measurement path with a rinsing liquid it performed. The output of the test measurement can, for instance, automatically trigger the execution of such a rinsing. It is also possible that the need of such a rinsing is indicated in one way or the other (e.g. by means of a display, a warning lamp or the like) and is then activated manually, e.g., by an applicant.

In one embodiment of the method according to the invention, which may be combined with any of the preaddressed embodiments and any of the embodiments still to be addressed unless in contradiction, the test measurement comprises measuring a refraction index, a pH-value, a conductivity, a density, a viscosity and /or an absorption of said reference substance.

A further aspect of the invention concerns a mobile maintenance device according to claim 12.

The mobile maintenance device according to the invention comprises a rollable support structure for flexibly moving towards and from a lubricant stored in a storage tank, an identifier configured to identify the lubricant stored in the storage tank, a computer storing data sets of different lubricants, each data set comprising at least one desired value of a parameter of the associated lubricant, and a measurement unit for determining at least one actual value of a parameter of said identified lubricant. Both the identifier and the measurement unit are operatively connected to inputs of the computer. The computer generates at an output status information of said identified lubricant, the status information being based on comparing said at least one actual value of said parameter of said identified lubricant with said at least one desired value of said parameter of said identified lubricant.

By using such a device, process safety can be increased, life time of tools and the (cooling) lubricant can be extended, operating cost can be minimized, and/or bacterial growth can be recognized at an early stage. The above described method of mobile maintenance survey for production sites with lubricant storage tanks can, for instance, be performed by means of such a device. The rollable support structure can be any trolley, carriage or the like being suitable to flexibly move the mobile maintenance device from and to a lubricant storage tank, preferably in a convenient manner. The identifier being part of the device can be, e.g., a barcode scanner or the like configured to scan an identification batch of the lubricant arranged at or close to the storage tank of said lubricant. The computer storing data sets of different lubricants can be a calculating unit, a computing machine, a laptop, a PC, a tablet, a phone or the like. The operative connection between the identifier and the computer can be implemented by wire or wireless. The same applies for the operative connection between the measurement unit and the computer. Consequently, the computer must not necessarily be attached to or placed on the rollable support structure or be anyhow nearby or in the surrounding of the other elements (such as the identifier, the measurement unit, ...) it is operatively connected to, but can physically be positioned anywhere, e.g., distant from the other components of the device. The measurement unit may comprise a measurement path and at least one sensor for determining at least one actual value of a parameter of the lubricant. The measurement unit may be operated directly by the computer of the device. The computer is also responsible for the generation of a status information. Said status information can be retrieved at an output of the computer and be displayed on a screen or the like.

In one embodiment of the device according to the invention, which may be combined with any of the embodiments still to be addressed unless in contradiction, the measurement unit comprises at least one sensor for determining at least pH, conductivity, density, temperature, viscosity, absorption, refractive index, total hardness, concentration of the lubricant concentrate, nitrite concentration, nitrate concentration, chloride concentration, metal concentration, such as cobalt and/or beryllium, and/or bacteria count. Furthermore, the at least one parameter can comprise a time series of one of the above identified measurands, a change rate of one of the above identified measurands and/or an interrelated combination of more than one of the above identified measurands.

Examples for such sensors are, for instance, pH meters, Brix sensors, thermometers and the like. Various methods such as sonic measurements, surface acoustic wave (SAW) measurements, refraction measurements and spectrometric measurements can be applied to measure the concentration of the lubricant concentrate. In case more than one sensor is implemented in the measurement unit, the sensors can be arranged in line along the measurement path. It is also possible to split the measurement path in several measurement sub-paths and thus arrange the sensors in parallel along the sub-paths. In case there are several sensors, but for the measurement to be performed it is not relevant to determine all the actual values that can be determined by means of all the sensors, the lubricant may bypass some of the sensors. For instance, some sub-paths can be blocked by a valve.

In one embodiment of the device according to the invention, which may be combined with any of the preaddressed embodiments and any of the embodiments still to be addressed unless in contradiction, the device further comprises an output medium for indicating said status information and/or for indicating the need of at least one additive in dependency of said status information.

The output media is preferably a screen, a lamp or a speaker. The output media can, e.g., be a smartphone, a tablet, or a PC of an applicant. The output media can be fixedly installed, e.g., at the rollable support structure, or removably attached, e.g., to the computer. However, the output media must not be physically attached or attachable to the mobile maintenance device at all (e.g. when the output media is a smart phone).

In one embodiment of the device according to the invention, which may be combined with any of the preaddressed embodiments and any of the embodiments still to be addressed unless in contradiction, the device further comprises a filling unit for automatically adding at least one additive to said identified lubricant in the storage tank in dependency of said status information. The filling unit comprising at least one reservoir comprising an additive for automatically being added to said identified lubricant in the storage tank in dependency of said status information.

For instance, the filling unit may execute the addition of water to the lubricant in the storage tank, when the status information indicates that the lubricant concentrate concentration is too high. For this purpose, the filling unit comprises a reservoir that is at least partially filled with water. In a preferred embodiment, the filling unit comprises at least two reservoirs, one for water and one for lubricant concentrate. In an even more preferred embodiment, the filling unit comprises a reservoir for water and various reservoirs for lubricants differing from each other. The filling unit may also comprise a mixer adapted to mix water and (one of the) lubricant(s) before these additives are actually added to the lubricant in the storage tank.

In one embodiment of the device according to the invention, which may be combined with any of the preaddressed embodiments and any of the embodiments still to be addressed unless in contradiction, the additive is at least one of a lubricant, of a lubricant concentrate, of water, and of a mixture thereof.

In one embodiment of the device according to the invention, which may be combined with any of the preaddressed embodiments and any of the embodiments still to be addressed unless in contradiction, at least one level sensor adapted to determine the filling level of said identified lubricant in the storage tank is operatively connected to an input of the computer.

For instance, the level sensor may send a signal to the computer indicating that it is not covered by lubricant anymore. The computer then processes said signal and generates status information indicating that the filling level of the storage tank is too low and/or that lubricant must be added. In case at least two level sensors are involved, one sensor may indicated the maximum filling level and the other sensor may indicate the minimum filling level. When the computer receives a signal indicating that the minimum level is reached, it can start the filling unit which is then adding lubricant to the storage tank. As soon as the computer receives a signal indicating that the maximum level is reached, it stops the filling unit and thus the addition of further lubricant.

In one embodiment of the device according to the invention, which may be combined with any of the preaddressed embodiments and any of the embodiments still to be addressed unless in contradiction, the device further comprises a power source, preferably a rechargeable power source, for driving the rollable support structure, the identifier, the computer, the measurement unit, and/or the filling unit.

As various of the components of the device need power to operate, the power must either be provided at or next to the lubricant storage tank(s) by means of an external power connector or the like, or by a power source being part of the maintenance device. Such an integrated power source can be an electric generator, a photovoltaic system or the like. However, a battery, in particular a rechargeable battery, is preferred.

In one embodiment of the device according to the invention, which may be combined with any of the preaddressed embodiments and any of the embodiments still to be addressed unless in contradiction, the device further comprises at least one feed line for supplying the measurement unit with the identified lubricant and/or at least one connector for connecting a feed line for a lubricant, the feed line being part of the storage tank.

In order to supply the measurement unit with the lubricant to be analyzed, the lubricant must somehow be transferred from the storage tank to the device or rather to the measurement unit. To do so, the device can be equipped with a hose adapted to be inserted into a lubricant storage tank and to transport an amount of lubricant from the tank to the measurement unit. The hose can be operatively connected to a pump. As an alternative or in addition, the device may comprise a connector adapted to connect to a hose belonging to a storage tank and leading from the inside of the storage tank to the outside. For instance, each storage tank to be surveyed may comprise such a hose. For performing the survey of several storage tanks, the mobile maintenance device must just be wheeled from one tank to the other and connected to the corresponding hose on site. One hose per storage tank also minimizes the risk of cross contamination compared to the use of one hose belonging to the mobile maintenance device and being used for all storage tanks.

In one embodiment of the device according to the invention, which may be combined with any of the preaddressed embodiments and any of the embodiments still to be addressed unless in contradiction, the computer is adapted to control the rollable support structure, receive and process information from the identifier, select a data set corresponding to said identified lubricant in dependency of the information from the identifier (the data set comprising at least one desired value of a parameter of the identified lubricant), control the measurement unit and receive and process information from the measurement unit (the information including at least one actual value of a parameter of said identified lubricant), compare said at least one actual value of said parameter of said identified lubricant with said at least one corresponding desired value of said parameter of said identified lubricant, and/or generate, depending on a result of said comparing, status information of said identified lubricant.

The computer can, for instance, optionally dictate the way along which the support structure rolls from and to a lubricant storage tank and/or initiates the start and stop of the movement of the support structure. The computer can also define several distances from one lubricant storage tank to another, join them to a sequence of distances and control the support structure such that it rolls along said defined sequences.

The invention shall now be further exemplified with the help of figures. The figures show:
Fig. 1 a flow chart of a method according to the invention of mobile maintenance survey for production sites with lubricant storage tanks;
Fig. 2 a schematic illustration of an embodiment of a method according to the invention of mobile maintenance survey for production sites with lubricant storage tanks;
Fig. 3 a schematic illustration of a mobile maintenance device according to the invention;
Fig. 4 a schematic illustration of an embodiment of a mobile maintenance device according to the invention;
Fig. 5 a schematic illustration of another embodiment of a mobile maintenance device according to the invention;
Fig. 6 a schematic illustration of a measurement unit as it can be implemented in a mobile maintenance device according to the invention;
Fig. 7 a schematic illustration of an embodiment of a measurement unit as it can be implemented in a mobile maintenance device according to the invention;
Fig. 8 a schematic illustration of another embodiment of a measurement unit as it can be implemented in a mobile maintenance device according to the invention;
Fig. 9 a schematic illustration of a measurement unit and a filling unit as implementable in a mobile maintenance device according to the invention; and
Fig. 10 a schematic illustration of a further embodiment of a measurement unit as it can be implemented in a mobile maintenance device according to the invention.

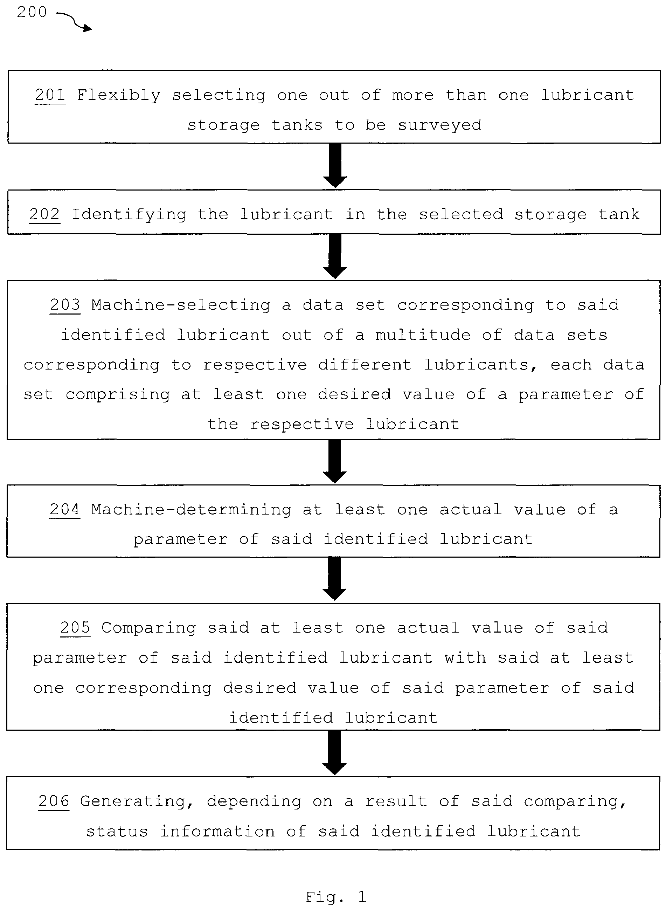

Fig. 1 shows a flow chart illustrating a method 200 of mobile maintenance survey for production sites with lubricant storage tanks. In a first step, one out of more than one lubricant storage tanks to be surveyed is flexibly selected 201. In a second step, the lubricant in the selected storage tank is identified 202. In a third step, a data set that corresponds to the identified lubricant is machine-selected out of a multitude of data sets corresponding to respective different lubricants 203. Each data set comprises at least one desired value of a parameter of the respective lubricant, i.e. the lubricant it corresponds to. In a next step, at least one actual value of a parameter of the identified lubricant is machine-determined 204. In a further step, the at least one actual value of the parameter of the identified lubricant is compared with the at least one corresponding desired value of the parameter of the identified lubricant 205. In an even further step, status information of the identified lubricant is generated, depending on a result of the foregoing comparing 206.

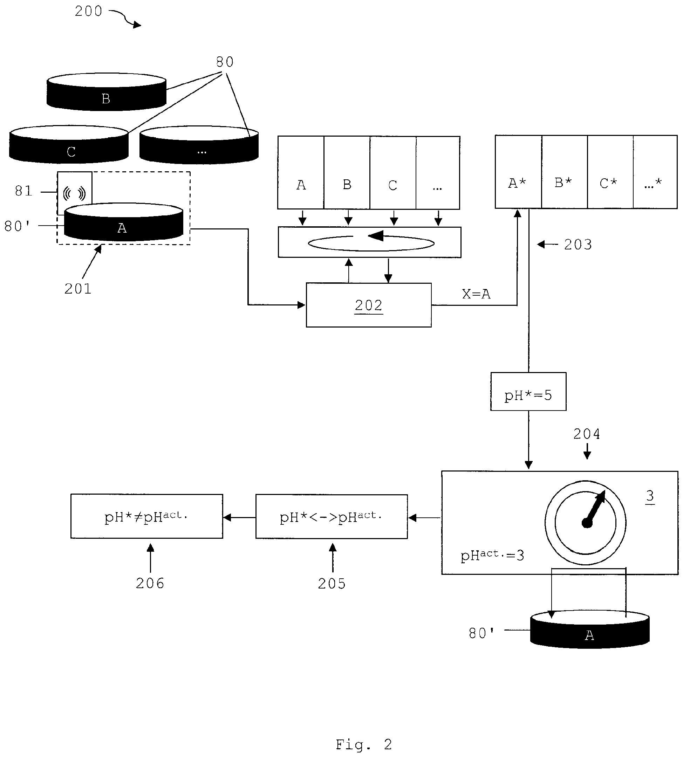

Fig. 2 shows a schematic illustration of an embodiment of a method 200 according to the invention. In a first step, one lubricant storage tank 80' is flexibly selected 201 out of more than one lubricant storage tanks 80. In order to identify 202 the lubricant in the selected storage tank 80', it is helpful if the storage tanks 80 are equipped with an identification symbol 81. Such an identification symbol 81 can be read by an identifier being part of a mobile maintenance device and assist in finding the proper data set. The mobile maintenance device may also comprise a computer that stores data sets of various lubricants (in the illustration labeled A, B, C, ...). An identifier receiving a signal depending on an identification symbol can send said signal to the computer, which then checks if there is a match with the lubricants stored in its memory. As soon as the lubricant A is identified 202, the computer selects 203 out of a multitude of data sets (in the illustration labeled A*, B*, C*,...) the data set that corresponds to the identified lubricant. Each data set comprises at least one desired value of a parameter of the identified lubricant. In the illustrated example, the desired value of a parameter being part of the data set is a pH-value, namely the pH* that is set to 5. To determine 204 at least one actual value of a parameter of the lubricant (in this example the actual value of a parameter is also a pH value, namely the pH^{act.}). In order to determine said value, a measurement unit 3 is supplied with an amount of lubricant of the selected storage tank 80' filled with lubricant A. After an actual pH value of 3 is machine-determined, the actual parameter pH^{act.} is compared 205 with the desired parameter pH*. Depending on said comparison, status information of the identified lubricant A is generated 206. In the illustrated example, the status information indicates that the actual value of the parameter pH^{act.} is not identic to the desired value of the parameter pH*.

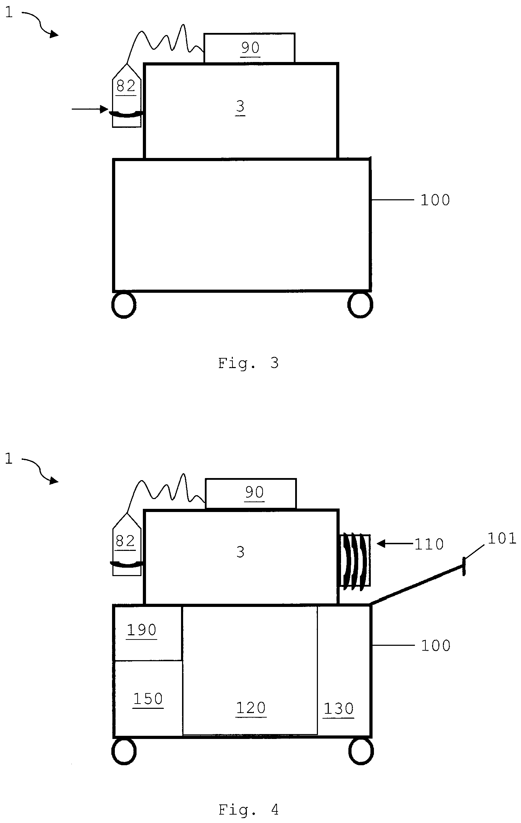

Fig. 3 schematically shows a mobile maintenance device 1 according to the invention. The mobile maintenance device 1 comprises a trolley 100 or any other rollable support structure for carrying, supporting and/or holding the maintenance device 1 and for flexibly moving the maintenance device 1 towards and from one lubricant storage tank to another. In the given example, the maintenance device 1 comprises an identifier 82 (e.g. removably attachable via a holder) to identify a lubricant stored in a storage tank and a computer 90 for storing data sets of different lubricants. The identifier 82 and the computer 90 are operatively connected (here shown by a wire; however, the connection can be wireless as well) and may communicate with each other such that the data set belonging to the lubricant that is identified by means of the identifier can be loaded automatically from a memory of the computer 90. The maintenance device 1 additionally comprises a measurement unit 3 that is also operatively connected to the computer 90 and can be operated by the computer 90. For instance, the computer 90 can control the measurement unit 3 such that it starts and stops a measurement, selects the measurement program to be run and also the parameters to be determined, initiates cleaning programs and reference measurements and so on. The computer 90 can also process signals from the measurement unit 3, calculate actual values of parameters of the lubricant based on the signals from the measurement unit 3 and compare the actual determined values of parameters with the desired values of parameters from the data set. Based on the comparison, the computer 90 can also generate status information that can, e.g., be displayed at a display or screen (not shown).

Fig. 4 schematically shows an embodiment of a mobile maintenance device 1 according to the invention. Apart from the features already described in connection with Fig. 3, the trolley 100 of this embodiment comprises a handle bar 101 for more convenient movement. In addition, the mobile maintenance device 1 comprises a hose 110 for aspirating some lubricant from the storage tank to feed the measurement unit 3 with lubricant. The hose 110 in this example is held by a cable drum allowing the easy unwinding and rewinding of the hose 110. Furthermore, the shown mobile maintenance device 1 comprises a waste tank 150 for collecting substances having, e.g., passed the measurement unit 3. The mobile maintenance device 1 also comprises a reservoir filled with water 120 to feed the measurement unit 3 with water and a reservoir filled with a cleaning agent 130 to feed the measurement unit 3 with cleaning agent. In case the mobile maintenance device 1 carries water and cleaning agent along, the measurement unit 3 can easily be cleaned between the maintenance of one lubricant and another lubricant. In this embodiment there is also a transportable power supply 190 in form of a (rechargeable) battery present for powering, e.g., the computer 90, the measurement unit 3 and so on.

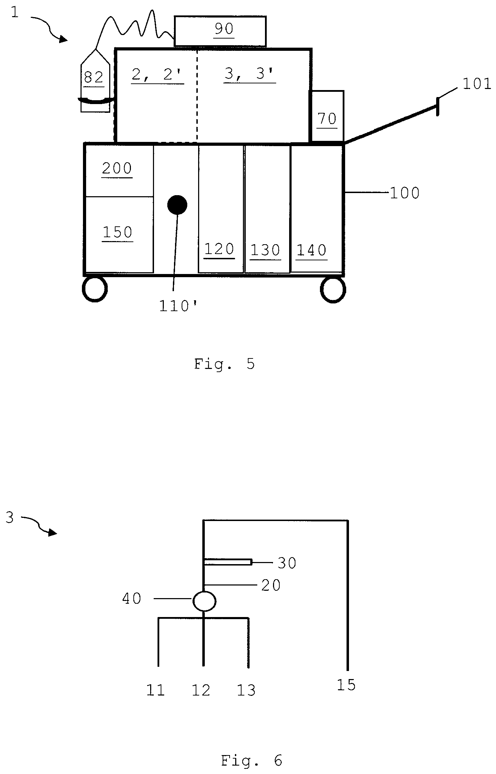

Fig. 5 schematically shows another example of a mobile maintenance device 1. This mobile maintenance device 1 is comparable to the one shown in Fig. 4, however, it comprises not only a measurement unit 3 but also a filling unit 2. The filling unit 2 and the measurement unit can either be designed as sub-units of one big unit or as separate units. For filling a storage tank not only with water but also with lubricant concentrate, the mobile maintenance device 1 comprises also a reservoir 140 filled with lubricant concentrate. Instead of the hose shown in Fig. 4, this mobile maintenance device 1 comprises a connector 110' for connecting a feed line for a lubricant, the feed line being part of the storage tank (e.g. the hose being permanently installed in the tank) in order to feed the measurement unit 3 with lubricant.

Fig. 6 schematically shows a measurement unit 3 as it can be implemented in a mobile maintenance device according to the invention. The measurement unit 3 comprises a first feed line 11, a second feed line 12 and a third feed line 13. The first feed line 11 supplies the measurement unit 3, e.g., with lubricant. The lubricant may be dispensed from a lubricant storage vessel (not shown), such as a tank, e.g., via a hose with a filer at is end. The second feed line 12 supplies the measurement unit 3, e.g., with water, such as tap water or purified water, either from a reservoir or directly from a tap. The water should be under pressure, such as usual line pressure of e.g. 2-6 bar. The third feed line 13 supplies the measurement unit 3, e.g., with one or more cleaning agents, in particular one or more liquid cleaning agents. The feed lines 11,12,13 merge to a joint line 20 either at one specific point, as shown here, or successively. The transportation of the lubricant, the water and the cleaning agent may be supported by a single pump 41 arranged at the joint line as shown here. However, each feed line 11,12,13 may comprise its own pump and the joint line 20 may then or may not comprise an additional pump. A sensor 30 for measuring a parameter of any of the substances passing through said joint line 20 is arranged along the joint line 20, either before (as shown) or after the sensor 30 (in supply or sucking direction leading from the first, second and third feed line 11,12,13 to the outlet 15 of the measurement unit 3, which outlet 15 is located after the sensor 30, e.g., in the form of an aperture).

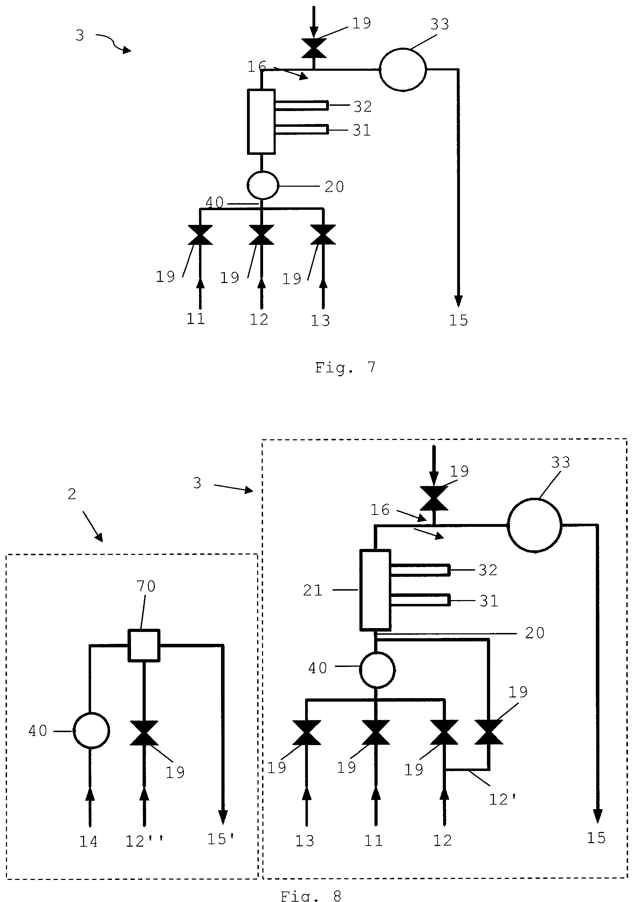

The measurement unit 3 schematically shown in Fig. 7, which measurement unit 3 can be implemented in a mobile maintenance device according to the invention, also comprises a first feed line 11, a second feed line 12 and a third feed line 13. Each of these feed lines 11,12,13 comprise a valve 19 to open and close the feed line. The joint line 20 comprise a tube-like section 21, where a first sensor 31 and a second sensor 32 are arranged at. After this tube-like section 21 (in supply or sucking direction, indicated by the arrows at the feed lines 11,12,13 and the outlet 15), a third sensor 33 is arranged. In case this third sensor 33 is a Brix sensor, it is advantageous to have a ventilation opening 16, also openable and closeable by means of a valve 19. Such ventilation opening 16 helps to free the Brix sensor from the liquid to be measured it was wetted with.

In Fig. 8 an embodiment of a measurement unit 3 and a separate filling unit 2 is schematically shown. Such a measurement unit 3 and separate filling unit 2 can be implemented in a mobile maintenance device according to the invention. Said filling unit 2 comprises a feed line 14 for a lubricant concentrate, a feed line 12'' for water, a mixer 70 for mixing the lubricant concentrate and the water, and an outlet 15'. The measurement unit 3 is identical to the measurement unit 3 of Fig. 7 apart from the additional feed line 12' leading from the second feed line 12 to the joint line 20, merging with said joint line 20 after the pump 40 and before the sensors 31,32 (in supply or sucking direction). In order to fully benefit from the filling unit 2, the lubricant storage tank (not shown) might be equipped with two sensors (not shown) in operative connection with the filling unit 2 and being capable of detecting the presence of the lubricant in the storage tank. One of the sensors represents the desired maximum level, the other sensor represents the desired minimum level of the lubricant in the storage tank. In case the lubricant level reaches its minimum, the "minimum sensor" triggers a refill or filling, e.g., directly via the filling unit 2 or via a computer controlling the filling unit 2. In combination with the result of a concentration measurement indicating the concentration of the lubricant concentrate in the lubricant gained by the measurement unit 3 and with the knowledge of the volume difference of the minimum and maximum level, the filling unit 2 can supply the storage tank via outlet 15' with the right amount of water and lubricant concentrate (optionally mixed by means of the mixer 70 before being filled into the storage tank) such that both the overall concentration of lubricant concentrate in the storage tank reaches its optimum and the storage tank is filled to its maximum level.

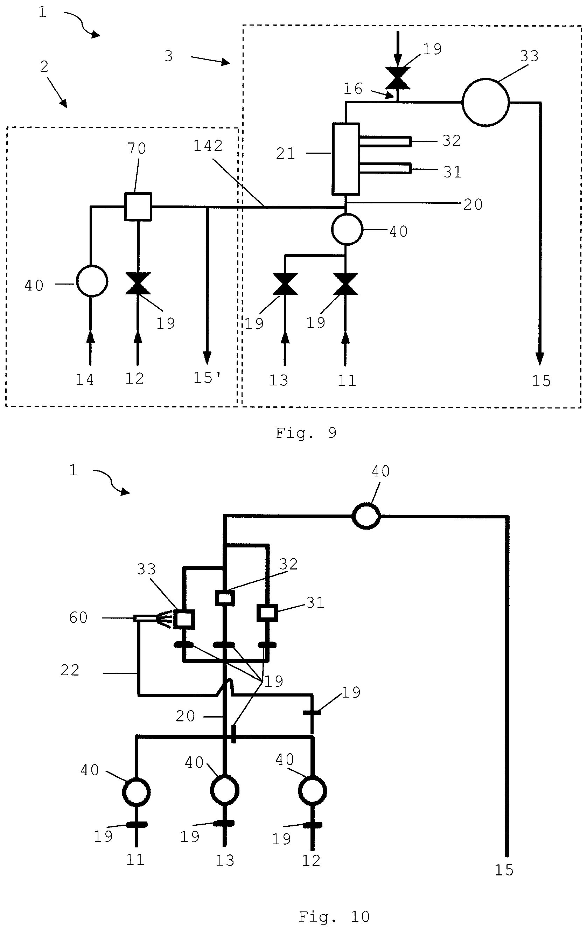

In Fig. 9 an embodiment of a measurement unit 3 and a filling unit 2 is schematically shown. Such a measurement unit 3 and filling unit 2 can be implemented in a mobile maintenance device according to the invention. In this embodiment the measurement unit 3 and the filling unit 2 are designed as a joint unit. The "integrated" filling unit 2 differs from the separate filling unit (e.g. shown in Fig. 8) in the feed line 142 that leads from the mixer 70 to the joint line 20 in order to merge with the first feed line 11 and the third feed line 13. The water supply for the mixer 70 and for the measurement unit 3 is provided by a single feed line, namely the second feed line 12. Consequently, the measurement unit 3 does not comprise an extra feed line for water as, for instance, the embodiment of Fig. 8 does.

Fig. 10 schematically shows another embodiment of a measurement unit 3, which could well be combined with a separate or integrate filling unit. Such a measurement unit 3 can be implemented in a mobile maintenance device according to the invention. The measurement unit 3 comprises a first feed line 11, e.g. for supplying a lubricant, a second feed line 12, e.g. for supplying water, and a third feed line 13, e.g. for supplying one or more cleaning agents. At each of the aforementioned feed lines 11,12,13 a valve 19 and a pump 40 are arranged. The first, second and third feed line 11,12,13 merge in a joint line 20. The measurement unit 3 comprises three sensors 31,32,33 that are supplied with lubricant or water and/or cleaning agent by the joint line 20. Said joint line 20 splits up in three branches or measurement sub-paths, each branch supplying one sensor, i.e. there is one sensor arranged at each branch of the joint line 20. Each branch can be regulated by means of a valve 19 such that a measurement must not be carried out by every sensor or rather that not every sensor must be brought into contact with the substance to be measured, although only certain measurement values are of interest. In order to maintain the good performance of sensors for as long as possible, it might be useful to clean the sensors not only by rinsing the measurement unit 3 with water or a (dilute) cleaning agent but also to direct a jet 60 on a sensor 33. The jet 60 can splash water or a (dilute) cleaning agent directly onto the sensor 33 and provide thus for a better cleaning result. The jet 60 can be supplied with water and/or (dilute) cleaning agent by a jet feed line 22 that might be connected to the second feed line 12, e.g. providing water, and the third feed line 13, e.g. providing cleaning agent(s). The supply of the jet 60 is then regulated via valves 19 that are, for instance, arranged around or next to the merging point of the joint line 20, i.e. the point where the first, second and third feed line 11,12,13 merge, and/or via a valve 19 that is arranged at the jet feed line 22. However, the jet feed line 22 might, as an alternative or in addition, be supplied by means of an external source and/or additional feed lines being part of the measurement unit. A further pump 40 is arranged between the sensors 31,32,33 and the outlet 15 of the joint line 20. Said pump 40 helps to transport the lubricant, water and (dilute) cleaning agent(s) though the measurement unit 3.

**Reference Signs**

| | |
|---|---|
| 1 | Mobile maintenance device |
| 2 | Filling unit |
| 3 | Measurement unit |
| 11 | First feed line |
| 12, 12', 12" | Second feed line |
| 13 | Third feed line |
| 14 | Feed line |
| 15, 15' | Outlet |
| 19 | Valve |
| 20 | Joint line |
| 21 | Tube |
| 22 | Jet feed line |
| 30 | Sensor |
| 31 | First sensor |
| 32 | Second sensor |
| 33 | Third sensor |
| 40 | Pump |
| 60 | Jet |
| 70 | Mixer |
| 80, 80' | Lubricant storage tank |
| 81 | Identification symbol |
| 82 | Identifier |
| 100 | Support structure |
| 101 | Handle bar |
| 110 | Hose |
| 110' | Connector |
| 120, 130 | Reservoirs comparing additives |
| 142 | Feed line |
| 150 | Waste tank |
| 190 | Power supply |
| 200 | Method |
| 201-206 | Steps of the method |

## Claims

1. Method of mobile maintenance survey for production sites with lubricant storage tanks, the method comprising:
- Flexibly selecting one out of more than one lubricant storage tanks to be surveyed;
- Identifying the lubricant in the selected storage tank;
- Machine-selecting a data set corresponding to said identified lubricant out of a multitude of data sets corresponding to respective different lubricants, each data set comprising at least one desired value of a parameter of the respective lubricant;
- Machine-determining at least one actual value of a parameter of said identified lubricant;
- Comparing said at least one actual value of said parameter of said identified lubricant with said at least one corresponding desired value of said parameter of said identified lubricant;
- Generating, depending on a result of said comparing, status information of said identified lubricant.

2. Method according to claim 1, wherein the parameter comprises at least one of:
- pH;
- conductivity;
- density;
- temperature;
- viscosity;
- absorption;
- refractive index;
- total hardness;
- concentration of the lubricant concentrate;
- nitrite concentration;
- nitrate concentration;
- chloride concentration;
- metal concentration;
- bacteria count;
- time series;
- change rate;
- interrelated combination.

3. Method according to claim 1 or 2, further comprising:
- Displaying the need of at least one additive in dependency of said status information; and/or
- Automatically adding at least one additive to said identified lubricant in the selected storage tank in dependency of said status information.

4. Method according to claim 3, wherein said additive is at least one of a lubricant, of a lubricant concentrate, of water, and of a mixture thereof.

5. Method according to one of claims 1 to 4, further comprising:
- Machine-determining the filling level of said identified lubricant in the selected storage tank; and
- Refilling the storage tank in dependency of the filling level.

6. Method according to one of claims 1 to 5, wherein said machine-determining of at least one actual value of a parameter of said identified lubricant comprises
performing a measurement.

7. Method according to claim 6, further comprising:
- Rinsing a measurement path for said measurement with a rinsing liquid comprising at least one of water and of a cleaning agent.

8. Method according to claim 6, wherein said rinsing is performed before and/or after flexibly selecting one out of more than one lubricant storage tanks.

9. Method according to one of claims 1 to 8, further comprising:
- Performing a test measurement with a reference substance, before said machine-determining of at least one actual value of a parameter of said identified lubricant.

10. Method according to one of claims 1 to 5, further comprising:
- Performing a test measurement with a reference substance, before said machine-determining of at least one actual value of a parameter of said identified lubricant; and
wherein said machine-determining comprises
performing a measurement and
rinsing a measurement path for said measurement with a rinsing liquid comprising at least one of water and of a cleaning agent,
said rinsing being performed in dependency of a result of said test measurement.

11. Method according to claim 9 or 10, wherein said test measurement comprises measuring at least one of a refraction index, of a pH-value, of a conductivity, of a density, of a viscosity and /or of an absorption of said reference substance.

12. Mobile maintenance device comprising:
- a rollable support structure for flexibly moving towards and from a lubricant stored in a storage tank;
- an identifier configured to identify the lubricant stored in the storage tank;
- a computer storing data sets of different lubricants, each data set comprising at least one desired value of a parameter of the associated lubricant;
- a measurement unit for determining at least one actual value of a parameter of said identified lubricant;
wherein both the identifier and the measurement unit are operatively connected to inputs of the computer, and wherein the computer generates at an output status information of said identified lubricant, the status information being based on comparing said at least one actual value of said parameter of said identified lubricant with said at least one desired value of said parameter of said identified lubricant.

13. Mobile maintenance device according to claim 12, wherein the measurement unit comprises at least one sensor for determining at least one of:
- pH-value;
- conductivity;
- density;
- temperature;
- viscosity;
- absorption;
- refractive index;
- total hardness;
- concentration of the lubricant concentrate;
- nitrite concentration;
- nitrate concentration;
- chloride concentration;
- metal concentration;
- bacteria count;
- time series;
- change rate;
- interrelated combination.

14. Mobile maintenance device according to claim 12 or 13, further comprising:
- an output medium, preferably a screen, a lamp or a speaker, for indicating said status information and/or for indicating the need of at least one additive in dependency of said status information.

15. Mobile maintenance device according to one of claims 12 to 14, further comprising:
- a filling unit for automatically adding at least one additive to said identified lubricant in the storage tank in dependency of said status information, the filling unit comprising at least one reservoir comprising an additive for automatically being added to said identified lubricant in the storage tank in dependency of said status information.

16. Mobile maintenance device according to claim 14 or 15, wherein said additive is at least one of a lubricant, of a lubricant concentrate, of water, and of a mixture thereof.

17. Mobile maintenance device according to one of the claims 12 to 16, wherein at least one level sensor adapted to determine the filling level of said identified lubricant in the storage tank is operatively connected to an input of the computer.

18. Mobile maintenance device according to one of the claims 12 to 17, further comprising:
- a power source, preferably a rechargeable power source, for driving at least one of the rollable support structure, of the identifier, of the computer, of the measurement unit, and of the filling unit.

19. Mobile maintenance device according to one of claims 12 to 18, further comprising:
- at least one feed line for supplying the measurement unit with the identified lubricant; and/or
- at least one connector for connecting a feed line for a lubricant, the feed line being part of the storage tank.

20. Mobile maintenance device according to one of claims 12 to 19, the computer being adapted to at least one of:
- Controlling the rollable support structure;
- Receiving and processing information from the identifier;
- Selecting a data set corresponding to said identified lubricant in dependency of the information from the identifier, the data set comprising at least one desired value of a parameter of the identified lubricant;
- Controlling the measurement unit and receiving and processing information from the measurement unit, the information including at least one actual value of a parameter of said identified lubricant;
- Comparing said at least one actual value of said parameter of said identified lubricant with said at least one corresponding desired value of said parameter of said identified lubricant;
- Generating, depending on a result of said comparing, status information of said identified lubricant.
